(19)

Europäisches Patentamt
European Patent Office
Office européen des brevets

(11) **EP 2 878 262 A1**

(12) **EUROPEAN PATENT APPLICATION**
published in accordance with Art. 153(4) EPC

(43) Date of publication:
**03.06.2015 Bulletin 2015/23**

(21) Application number: **13822814.3**

(22) Date of filing: **24.07.2013**

(51) Int Cl.:
*A61B 5/18* (2006.01)     *A61B 5/16* (2006.01)
*B60K 28/06* (2006.01)    *B60W 30/08* (2012.01)
*B60W 50/08* (2012.01)

(86) International application number:
**PCT/JP2013/004512**

(87) International publication number:
**WO 2014/017090 (30.01.2014 Gazette 2014/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **26.07.2012 JP 2012166227**

(71) Applicant: **Nissan Motor Co., Ltd.**
**Yokohama-shi**
**Kanagawa 221-0023 (JP)**

(72) Inventors:
• **SHIMIZU, Youji**
  **Atsugi-shi**
  **Kanagawa 243-0123 (JP)**

• **SUNDA, Takashi**
  **Atsugi-shi**
  **Kanagawa 243-0123 (JP)**
• **MORI, Souichiro**
  **Atsugi-shi**
  **Kanagawa 243-0123 (JP)**
• **MIURA, Hiroaki**
  **Atsugi-shi**
  **Kanagawa 243-0123 (JP)**
• **FUKUYAMA, Yasuhiro**
  **Atsugi-shi**
  **Kanagawa 243-0123 (JP)**

(74) Representative: **Osha Liang**
**2, rue de la Paix**
**75002 Paris (FR)**

(54) **DRIVER FATIGUE-ESTIMATING DEVICE AND DRIVER FATIGUE-ESTIMATING METHOD**

(57)     A driver's fatigue degree estimation apparatus includes a biological information value acquisition unit (2) configured to acquire a biological information value indicative of biological information of a driver of a vehicle, a vehicle speed detection unit (4) configured to detect a vehicle speed of the vehicle, a stable value convergence change degree detection unit (20) configured to detect a stable value convergence change degree which is a degree of change in the biological information value from a peak value of the biological information value increasing after the vehicle starts deceleration compared to the biological information value before the vehicle starts the deceleration, to a preset stable value, to which the biological information value decreases after the vehicle stops, based on the biological information value acquired by the biological information value acquisition unit (2) and the vehicle speed detected by the vehicle speed detection unit (4), and a fatigue degree estimation unit (22) configured to estimate a fatigue degree of the driver depending on the stable value convergence change degree detected by the stable value convergence change degree detection unit (20).

FIG. 1

# Description

Technical Field

This disclosure relates to a driver's fatigue degree estimation apparatus and a driver's fatigue degree estimation method for estimating a fatigue degree of a driver while driving.

Background Art

As a technique of estimating a fatigue degree of a driver while driving, for example, a technique described in PTL 1 is known. The technique described in PTL 1 detects a driving state, a driving pattern and a body condition of the driver, and comprehensively determines the fatigue degree, a consciousness degree and a concentration degree of the driver on the basis of these detection results. Then, the technique described in PTL 1 detects the reactivity degree of the driver on the basis of the above-mentioned various determination results, and controls the timing of alerting the driver on the basis of the detection result.

In this case, the body condition of the driver is detected by acquiring a biological information value indicative of biological information of the driver, such as a body temperature, an amount of sweating, a pulse rate or the like of the driver with a sensor provided on a steering wheel or the like.

Citation List

Patent Literature

PTL1: JP 2007-38911 A

Summary of Invention

Technical Problem

The above-mentioned biological information values of the driver may vary depending on factors in a driving situation such as a road environment, a traffic environment, changes in a driving load due to these environments, or the like, regardless of the fatigue degree. Therefore, in the technique described in PTL 1, a problem that the biological information values of the driver which vary due to the factor in the driving situation degrade estimate accuracy of the fatigue degree may occur.

The present invention has been made in view of the above problem, and has an object to provide a fatigue degree estimation apparatus and a driver's fatigue degree estimation method capable of suppressing the degradation of the estimate accuracy of the fatigue degree.

Solution to Problem

In order to solve the above-mentioned problem, according to an aspect of the present disclosure, a stable value convergence change degree is detected. The stable value convergence change degree is a degree of change in a biological information value from a peak value of the biological information value increasing after a vehicle starts deceleration compared to the biological information value before the vehicle starts the deceleration, to a preset stable value, to which the biological information value decreases after the vehicle stops. In addition, the fatigue degree of a driver may be estimated depending on the stable value convergence change degree detected in this way. In this case, the stable value convergence change degree may be detected based on the biological information value indicative of biological information of the driver of the vehicle and a vehicle speed of the vehicle.

Advantageous Effects of Invention

According to an aspect of the present disclosure, it is possible to estimate the fatigue degree of the driver based on the degree of change in the biological information value from the peak value of the biological information value increasing after the vehicle starts the deceleration compared to the biological information value before the vehicle starts the deceleration, to the preset stable value, to which the biological information value decreases after the vehicle stops.

Therefore, it is possible to estimate the fatigue degree of the driver by using the biological information values of the driver at the times when the vehicle decelerates and stops regardless of the driving situation in regard to the road environment, the traffic environment, or the like, and thus, it is possible to suppress the degradation of the estimate accuracy of the fatigue degree.

Brief Description of Drawings

FIG. 1 is a view illustrating a schematic configuration of a driver's fatigue degree estimation apparatus according to a first embodiment of the present invention;

FIG. 2 is a view illustrating a temporal change of a speed of a vehicle (vehicle speed) driven by a driver and a temporal change of a blood-pressure value of the driver;

FIG. 3A and FIG. 3B are views illustrating verification results in which the relationship between a deceleration stabilizing time and a deceleration stabilizing change amount is verified by experiments;

FIG. 4 is a flowchart illustrating one example of a processing performed by a driver's fatigue degree estimation apparatus; and

FIG. 5 is a flowchart illustrating one example of a processing performed by a modification of a driver's fatigue degree estimation apparatus.

Description of Embodiments

Hereinafter, embodiments of the present invention will now be described with reference to the drawings.

(First Embodiment)

Hereinafter, the first embodiment of the present invention (hereinafter, referred to as the present embodiment) will now be described with reference to the drawings.

(Configuration)

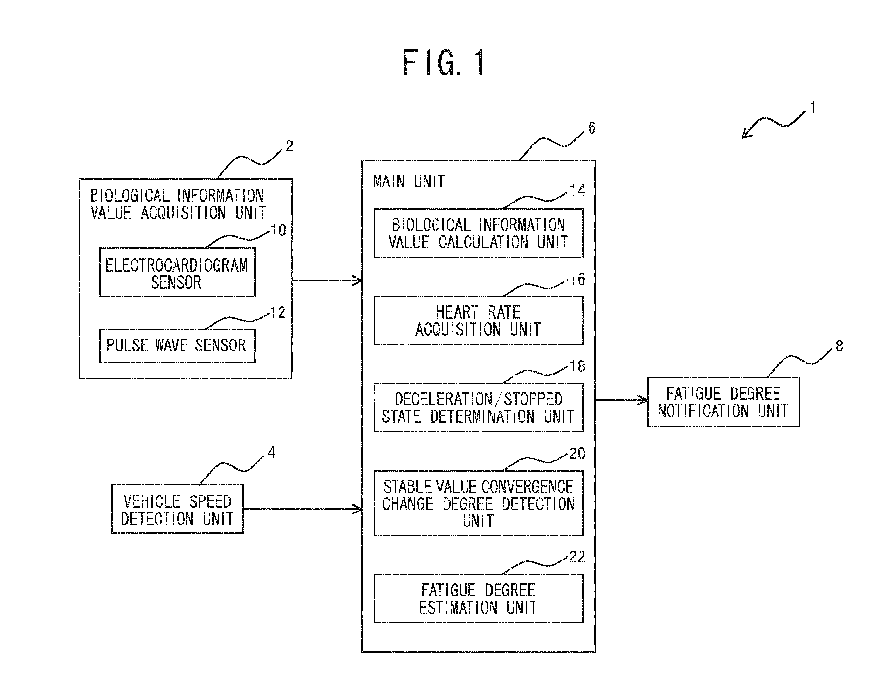

FIG. 1 is a view illustrating a schematic configuration of a driver's fatigue degree estimation apparatus according to the present embodiment.
As illustrated in FIG. 1, the driver's fatigue degree estimation apparatus 1 includes a biological information value acquisition unit 2, a vehicle speed detection unit 4, a main unit 6, and a fatigue degree notification unit 8.
The biological information value acquisition unit 2 includes an electrocardiogram sensor 10 and a pulse wave sensor 12.
The electrocardiogram sensor 10 is provided on, for example, a driver's seat of the vehicle not illustrated (hereinafter, may be referred to as "vehicle") including the driver's fatigue degree estimation apparatus 1, and is configured to detect an electrocardiographic waveform of the driver seated on the driver's seat. Then, the electrocardiogram sensor 10 is configured to output an information signal (hereinafter, may be referred to as "electrocardiographic waveform signal") including the detected electrocardiographic waveform to the main unit 6. It is noted that the installation location of the electrocardiogram sensor 10 is not limited to the driver's seat, and may be a grip portion of a steering wheel of the vehicle which the driver grips, for example.
In the present embodiment, the electrocardiogram sensor 10 may be formed to include plural electrodes provided on a seating face, a surface of a backrest of the seat, or the like. Then, the electrocardiogram sensor 10 is configured to detect a potential difference signal produced between the respective electrodes, and to detect an electrocardiographic waveform on the basis of the detected potential difference signal. It is noted that the potential difference signal produced between the respective electrodes due to a depolarization produced with a conduction of impulse which occurs when the heart muscle of the driver expands and contracts. Additionally, since clothes or a cloth is present between a skin of the driver and the electrodes, the electrocardiogram sensor 10 may have a configuration employing the Laplacian electrode arrangement or a configuration having a capacitive measurement circuit with a high input impedance.
The pulse wave sensor 12 is provided on, for example, the grip portion of the steering wheel of the vehicle which the driver grips and is configured to detect the pulse wave (volume pulse wave) of the driver. Then, the pulse wave sensor 12 is configured to output an information signal (hereinafter, may be referred to as "pulse wave signal") including the detected pulse wave to the main unit 6. It is noted that the installation location of the pulse wave sensor 12 is not limited to the grip portion of the steering wheel and may be the driver's seat, for example.
Herein, the pulse wave is the biological information representing a change of a volume of the peripheral arterial vessel associated with the pulsation of the heart. Therefore, the pulse wave sensor 12 may be formed by using a photoelectric sensor having a combination of a light emitting element and a light receiving element of the infrared light to utilize a characteristic of the near-infrared wavelength light passing through a living body and being absorbed into hemoglobin in blood. Specifically, the pulse wave sensor 12 is configured to irradiate the infrared light to a palm or a fingertip of the driver and detect a voltage signal depending on a light intensity of a scattered light that is reflected. Then, the pulse wave sensor 12 is configured to detect the pulse wave on the basis of the detected voltage signal.
The vehicle speed detection unit 4 is formed as a vehicle speed sensor, for example.
Furthermore, the vehicle speed detection unit 4 is configured to detect the current speed of the vehicle by using the rotation rate of the wheel included in the vehicle or the like. Then, the vehicle speed detection unit 4 is configured to output an information signal (hereinafter, may be referred to as "vehicle speed signal") including the detected current speed (hereinafter, may be referred to as "vehicle speed") to the main unit 6.
The main unit 6 includes a biological information value calculation unit 14, a heart rate acquisition unit 16, a deceleration/stopped state determination unit 18, a stable value convergence change degree detection unit 20, and a fatigue degree estimation unit 22.
The biological information value calculation unit 14 is configured to calculate a blood-pressure value of the driver as the biological information value indicative of the biological information of the driver, on the basis of the electrocardiographic waveform signal received from the electrocardiogram sensor 10 and the pulse wave signal received from the pulse wave sensor 12. Then, the biological information value calculation unit 14 is configured to output an information signal (hereinafter, may be referred to as "blood-pressure value signal") including the calculated blood-pressure value to the stable value convergence change degree detection unit 20.
Specifically, the biological information value calculation unit 14 is configured to calculate the blood-pressure value of the driver on the basis of the electrocardiographic waveform signal and the pulse wave signal by using the pulse transmission time method in accordance with the following expression (1).

$$BP = \alpha1 \cdot T_{PAT} + \beta1 \quad ... \quad (1)$$

It is noted that "BP" denotes the blood-pressure value and "$T_{PAT}$" denotes a pulse transmission time in the expression (1). Furthermore, $\alpha1$ and $\beta1$ in the expression (1) denote correction values specific to the driver. $\alpha1$ and $\beta1$ may be preset by experiments for example.

Furthermore, the pulse transmission time $T_{PAT}$ is calculated by the following expression (2).

$$T_{PAT} = Tb - Ta \quad ... \quad (2)$$

It is noted that, in the expression (2), "Ta" denotes a time when an R wave peak value appears in the electrocardiographic waveform included in the electrocardiographic waveform signal. Furthermore, "Tb" corresponds to a heart pumping timing, and denotes a time when the pulse waveform rises. "Tb" also corresponds to a time when the pulse wave arrives at a fingertip.

As mentioned above, in the present embodiment, there is described as an example, a case where the biological information value acquisition unit 2 is configured to acquire the blood-pressure value of the driver as the biological information value indicative of the biological information of the driver of the vehicle.

The heart rate acquisition unit 16 is configured to acquire the heart rate of the driver on the basis of the pulse wave signal received from the pulse wave sensor 12. Then, the heart rate acquisition unit 16 is configured to output an information signal (hereinafter, may be referred to as "heart rate signal") including the acquired heart rate to the fatigue degree estimation unit 22.

The deceleration/stopped state determination unit 18 is configured to determine whether or not the vehicle is in a deceleration state and whether or not the vehicle shifts from the deceleration state to a stopped state on the basis of the vehicle speed signal received from the vehicle speed detection unit 4. Then, the deceleration/stopped state determination unit 18 is configured to output an information signal (hereinafter, may be referred to as "determination result signal") including the determination result to the stable value convergence change degree detection unit 20.

It is noted that the deceleration/stopped state determination unit 18 may have a configuration to determine the state of the vehicle (the deceleration state, the shift from the deceleration state to the stopped state) by reference to, for example, an operation amount (depression amount) of a brake pedal by the driver in addition to the vehicle speed signal. In this case, a brake pedal sensor may be simultaneously used to detect the operation amount of the brake pedal by the driver, for example, and the state of the vehicle may be determined by detecting that the operation amount of the brake pedal is a value corresponding to a braking operation.

The stable value convergence change degree detection unit 20 is configured to detect the stable value convergence change degree on the basis of the blood-pressure value signal received from the biological information value calculation unit 14 and the determination result signal received from the deceleration/stopped state determination unit 18. Then, the stable value convergence change degree detection unit 20 is configured to output an information signal (hereinafter, may be referred to as "stable value convergence change degree signal") including the stable value convergence change degree detected in this way to the fatigue degree estimation unit 22.

Herein, the stable value convergence change degree is a degree of change in the biological information value (blood-pressure value) from a peak value of the biological information value increasing after the vehicle starts deceleration compared to the biological information value before the vehicle starts the deceleration, to a preset stable value, to which the biological information value decreases after the vehicle stops. It is noted that the peak value of the biological information value may be a maximum value of the biological information value. Furthermore, for example, when the biological information value fluctuates greatly or the like, an average value in an interval in which the biological information value keeps a similar value may be used.

As mentioned above, in the present embodiment, the biological information value calculation unit 14 is configured to calculate the blood-pressure of the driver as the biological information value of the driver. Therefore, there is described in the present embodiment, a case where the stable value convergence change degree detection unit 20 is configured to detect a blood-pressure convergence change degree as the stable value convergence change degree. Herein, the blood-pressure convergence change degree is a degree of change in the blood-pressure value from a maximum blood-pressure value of the blood-pressure value rising after the vehicle starts the deceleration compared to the blood-pressure value before the vehicle starts the deceleration, to a preset stable blood-pressure value, to which the blood-pressure value drops after the vehicle stops. It is noted that the maximum blood-pressure value corresponds to the peak value of the biological information value described above. Thus, the maximum blood-pressure value may be a maximum value of the blood-pressure value. Furthermore, when the blood-pressure value fluctuates greatly or the like, an average value in an interval in which the blood-pressure value keeps a similar value may be used.

Specifically, the stable value convergence change degree detection unit 20 is configured to always detect the change amounts of the blood-pressure value on the basis of the blood-pressure value signal received from the biological information value calculation unit 14 and accumulate the change amounts. In addition, the stable value convergence change degree detection unit 20 is configured to detect the deceleration state of the vehicle on the basis of the determination result signal received from the deceleration/stopped state determination unit 18.

Then, the stable value convergence change degree detection unit 20 is configured to compare the blood-pressure value detected after the vehicle starts the deceleration with the blood-pressure value detected before the vehicle starts the deceleration when the traveling vehicle is in the deceleration state, and detect the maximum blood-pressure value which is larger than the blood-pressure value before the vehicle starts the deceleration and is a maximum value, from the blood-pressure values detected after the vehicle starts the deceleration. Furthermore, the stable value convergence change degree detection unit 20 is configured to start a timer operation to start measurement of time at the time when the maximum blood-pressure value which is larger than the blood-pressure value before the vehicle starts the deceleration and is a maximum value, is detected from the blood-pressure values detected after the vehicle starts the deceleration.

Next, the stable value convergence change degree detection unit 20 is configured to determine whether or not the blood-pressure value drops from the maximum blood-pressure values and reaches the stable blood-pressure value when the traveling vehicle shifts from the deceleration state to the stopped state (a state in which the vehicle speed is 0 (km/h)).

Herein, for example, an average value of the blood-pressure values detected in a travelling state other than the deceleration state and the stopped state may be used as the stable blood-pressure value. Furthermore, the travelling state other than the deceleration state and the stopped state may be an acceleration state, a state in which the vehicle travels at a speed within a certain range, or the like. It is noted that the stable blood-pressure value may be set as a single value or as a range based on an upper limit and a lower limit of the interval in which the blood-pressure value keeps a similar value.

Then the stable value convergence change degree detection unit 20 is configured, when it is determined that the vehicle shifts from the deceleration state to the stopped state and the blood-pressure value reaches the stable blood-pressure value, to stop the above-mentioned timer operation to stop the measurement of time at the time when it is determined that the blood-pressure value reaches the stable blood-pressure value. The stable value convergence change degree detection unit 20 is configured to calculate a deceleration stabilizing time. The deceleration stabilizing time is an elapsed time from when the blood-pressure value starts to drop from the a maximum blood-pressure value of the blood-pressure value rising after the vehicle starts the deceleration compared to the biological information value before the vehicle starts the deceleration, to when the blood-pressure value reaches the stable blood-pressure value after the vehicle stops.

Furthermore, the stable value convergence change degree detection unit 20 calculates a deceleration stabilizing change amount on the basis of a difference between the maximum blood-pressure value and the stable blood-pressure value. Herein, the deceleration stabilizing change amount is a change amount in the blood-pressure value from a maximum blood-pressure value of the blood-pressure value rising after the vehicle starts the deceleration compared to the blood-pressure value before the vehicle starts the deceleration, to the stable blood-pressure value, to which the blood-pressure value drops after the vehicle stops.

The stable value convergence change degree detection unit 20, which calculates the deceleration stabilizing time and the deceleration stabilizing change amount, is configured to calculate a deceleration stabilizing change rate on the basis of the deceleration stabilizing time and the deceleration stabilizing change amount. The deceleration stabilizing change rate is a change rate of the blood-pressure value. Then, the stable value convergence change degree detection unit 20 is configured to detect the deceleration stabilizing change rate calculated in this way as the above-mentioned blood-pressure convergence change degree (stable value convergence change degree).

As mentioned above, in the present embodiment, there is described as an example, a case where the stable value convergence change degree detection unit 20 is configured to detect the blood-pressure convergence change degree as the stable value convergence change degree on the basis of the blood-pressure value of the driver acquired by the biological information value acquisition unit 2 and the vehicle speed detected by the vehicle speed detection unit 4.

The fatigue degree estimation unit 22 is configured to estimate the fatigue degree of the driver depending on the stable value convergence change degree (the blood-pressure convergence change degree) included in the stable value convergence change degree signal when receiving the stable value convergence change degree signal from the stable value convergence change degree detection unit 20. Then, the fatigue degree estimation unit 22 is configured to output a notice command signal generated beforehand to the fatigue degree notice unit 8 depending on a magnitude of the estimated fatigue degree.

Herein, the notice command signal generated by the fatigue degree estimation unit 22 is, for example, a signal for notifying information indicative of a message encouraging the driver to take a break from the fatigue degree notice unit 8, or a signal to invite attention of the driver. Additionally, the fatigue degree estimation unit 22 may be configured to output the notice command signal to the fatigue degree notice unit 8, for example, at the time when the magnitude of the fatigue degree estimated depending on the stable value convergence change degree is determined as exceeding a preset fatigue degree threshold. It is noted that the fatigue degree threshold may be a value corresponding to the stable value convergence change degree in a case where the driving time of the driver is about two hours, for example, and is calculated and set by experiments beforehand.

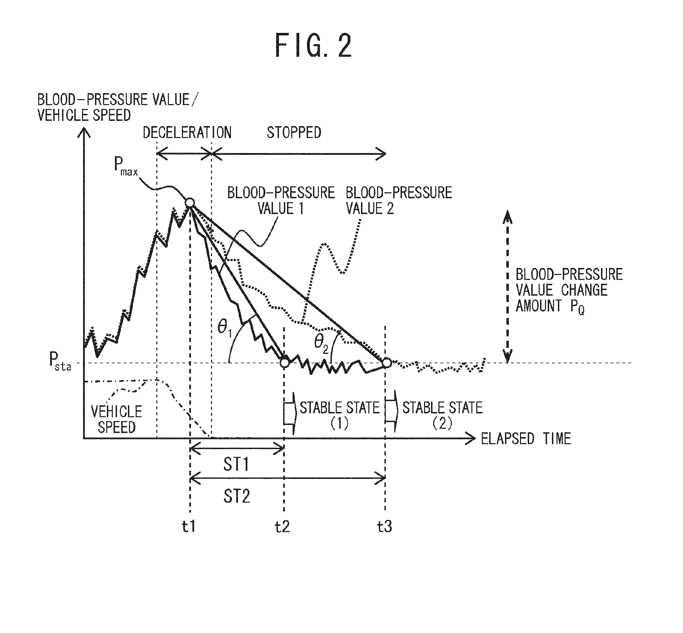

Hereinafter, an example of the processing for estimating the fatigue degree by the fatigue degree estimation unit 22 will be described with reference to FIG. 1 by using FIG. 2. It is noted that FIG. 2 is a view illustrating a temporal change of the speed of the vehicle (vehicle speed) driven by the driver and a temporal change of the blood-pressure value of the driver. FIG. 2 represents the elapsed time in abscissa and the vehicle speed and the blood-pressure value of the driver in ordinate.

When the fatigue degree estimation unit 22 performs a processing of estimation the fatigue degree, as illustrated in FIG. 2, the magnitude of the stable value convergence change degree detected by the stable value convergence change degree detection unit 20 is referred to firstly.

As illustrated in FIG. 2, the blood-pressure value varies (rises and drops) with pulsation. Then the travelling vehicle decelerates (the interval illustrated as "deceleration" in the drawing), the blood-pressure value rises with a decrease in the vehicle speed and reaches the maximum blood-pressure value $P_{max}$. It is noted that the vehicle speed is indicated with a chain line in FIG. 2.

In this case, the stable value convergence change degree detection unit 20 calculates the deceleration stabilizing time when the blood-pressure reaches the maximum blood-pressure value $P_{max}$, and then drops from the maximum blood-pressure value $P_{max}$ to the stable blood-pressure value $P_{sta}$. That is, the stable value convergence change degree detection unit 20 starts the above-mentioned timer operation from the time "t1" illustrated in the drawing and stops the timer operation when the vehicle shifts from the deceleration state to the stopped state (the interval illustrated as "stopped" in the drawing) and it is determined that the blood-pressure value reaches the stable blood-pressure value $P_{sta}$.

In this case, in order to describe the processing for estimating the fatigue degree by the fatigue degree estimation unit 22, FIG. 2 illustrates two examples as an example indicative of a change of the blood-pressure when the blood-pressure drops from the maximum blood-pressure value $P_{max}$ to the stable blood-pressure value $P_{sta}$.

One of the two examples indicates the change of the blood-pressure in a case where the blood-pressure drops from the time t1 when the blood-pressure reaches the maximum blood-pressure value $P_{max}$ and reaches the stable blood-pressure value $P_{sta}$ at the time "t2" illustrated in the drawing in the stopped state. It is noted that the blood-pressure value in the one of the two examples is indicated with a solid line in FIG. 2. Furthermore, the elapsed time from the time t1 to the time t2 is indicated as "ST1" in FIG. 2. Furthermore, in the one of the two examples in FIG. 2, the interval indicating the blood-pressure value after the blood-pressure reaches the stable blood-pressure value $P_{sta}$ at the time t2 is indicated as "stable state (1)".

Furthermore, the other of the two examples indicates the change of the blood-pressure in a case where the blood-pressure drops from the time t1 when the blood-pressure reaches the maximum blood-pressure value $P_{max}$ and reaches the stable blood-pressure value $P_{sta}$ at the time "t3" illustrated in the drawing, which is later than the time t2, in the stopped state. It is noted that the blood-pressure value in the other of the two examples is indicated with a dotted line in FIG. 2. Furthermore, the elapsed time from the time t1 to the time t3 is indicated as "ST2" in FIG. 2. Furthermore, in the other of the two examples in FIG. 2, the interval indicating the blood-pressure value after the blood-pressure reaches the stable blood-pressure value $P_{sta}$ at the time t3 is indicated as "stable state (2)".

In this case, the time t2 is earlier than the time t3, and the elapsed time ST1 is shorter than the elapsed time ST2, as illustrated in FIG. 2. Furthermore, the differences ("blood-pressure value change amount $P_Q$" illustrated in the drawing) between the maximum blood-pressure value $P_{max}$ and the stable blood-pressure value $P_{sta}$ in the two example are the same.

Additionally, each of the elapsed time ST1 and the elapsed time ST2 are an elapsed time while the blood-pressure value, which reaches a maximum value (maximum blood-pressure value $P_{max}$) after the vehicle starts the deceleration, changes to the stable value (stable blood-pressure value $P_{sta}$) after the vehicle stops, that is, the above-mentioned deceleration stabilizing time. Furthermore, the blood-pressure value change amount $P_Q$ is a change amount in the blood-pressure value while the blood-pressure value, which reaches the maximum blood-pressure value $P_{max}$ after the vehicle starts the deceleration, changes to the stable value (stable blood-pressure value $P_{sta}$) after the vehicle stops, that is, the above-mentioned deceleration stabilizing change amount.

Therefore, as illustrated in FIG. 2, the slope θ indicative of a change amount of the blood-pressure value relative to the elapsed time ST (ST1, ST2) in which the blood-pressure value varies from the maximum blood-pressure value $P_{max}$ to the stable blood-pressure value $P_{sta}$ represents the deceleration stabilizing change rate (stable value convergence change degree) as the change rate of the blood-pressure value.

As mentioned above, since the elapsed time ST1 is shorter than the elapsed time ST2, the slope θ1 indicative of a change amount of the blood-pressure value relative to the elapsed time ST1 is larger then the slope θ2 indicative of a change amount of the blood-pressure value relative to the elapsed time ST2.

Furthermore, the fatigue degree estimation unit 22 in the present embodiment is configured to estimate that the fatigue degree of the driver is larger, as the stable value convergence change degree is smaller.

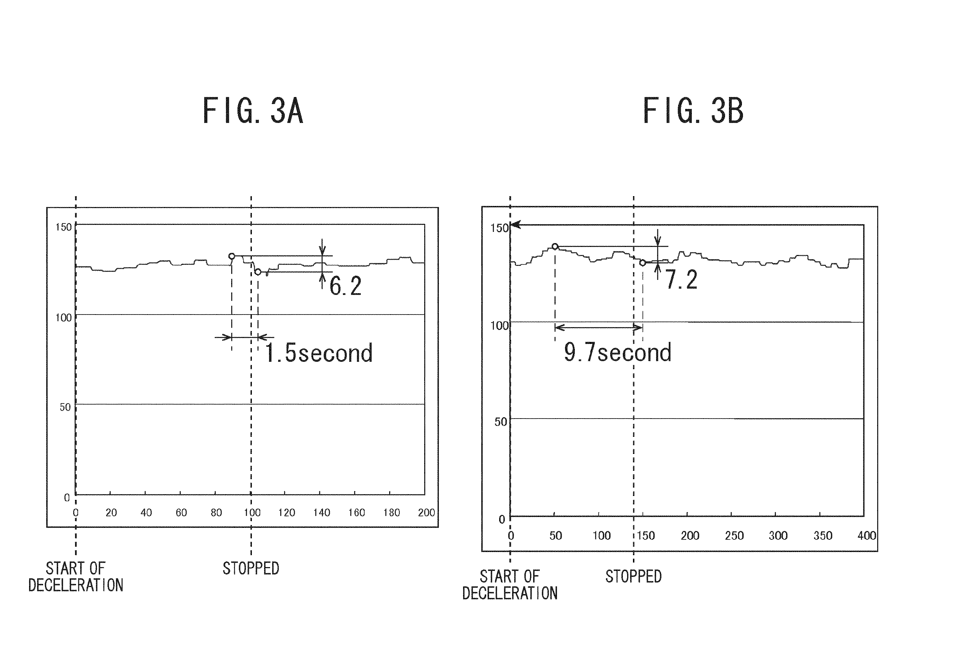

Hereinafter, the relationship between the stable value convergence change degree and the fatigue degree of the driver will be described with reference to FIG. 1 and FIG. 2 by using FIG. 3A and FIG. 3B. It is noted that FIG. 3A and FIG. 3B are views illustrating verification results in which the relationship between the deceleration stabilizing time and the deceleration stabilizing change amount is verified by experiments. FIG. 3A and FIG. 3B are different from each other in the driving time of the driver. Specifically, FIG. 3A illustrates the verification result in a case where the driving time is shorter than that in the verification result illustrated in FIG. 3B. Furthermore, FIG. 3A and FIG. 3B represent the elapsed time in abscissa and the blood-pressure value of the driver in ordinate.

As illustrated in FIG. 3A and FIG. 3B, in a state in which it is estimated that the driving time of the driver is long, that is, the fatigue degree of the driver is large, the blood-pressure change amount per one second is reduced and the deceleration stabilizing time is extended.

Specifically, in a state in which the driving time (an elapsed time from the start of the experiment) is fifteen minutes as illustrated in FIG. 3A, the deceleration stabilizing change amount is 6.2 and the deceleration stabilizing time is 1.5 seconds. In contrast, in a state in which the driving time is five hours as illustrated in FIG. 3B, the deceleration stabilizing change amount is 7.2 and the deceleration stabilizing time is 9.7 seconds.

Therefore, in the state in which the driving time is fifteen minutes, the blood-pressure change amount per one second is about 4.13. In contrast, in the state in which the driving time is five hours, the blood-pressure change amount per one second is about 0.74.

Therefore, the state in which the deceleration stabilizing time is extended for the same or a similar deceleration stabilizing change amount can be said as a state in which the fatigue degree of the driver is estimated as being large such as a state in which the driving time of the driver becomes longer.

For the reasons stated above, the fatigue degree estimation unit 22 of the present embodiment is configured to estimate that the fatigue degree of the driver is larger, as the slope indicative of the change amount of the blood-pressure value relative to the elapsed time is smaller, that is, as the elapsed time in which the blood-pressure value varies from the maximum blood-pressure value $P_{max}$ to the stable blood-pressure value $P_{sta}$ is longer. Therefore, in the examples illustrated in FIG. 2, it is estimated that the fatigue degree of the driver is large when the stable value convergence change degree detection unit 20 detects the stable value convergence change degree of the blood-pressure value indicated in dotted line in the drawing out of the two examples.

This completes the description of the processing for estimating the fatigue degree by the fatigue degree estimation unit 22.

Furthermore, the fatigue degree estimation unit 22 is configured to compare the heart rate included in the heart rate signal with a preset heart rate stable value on the basis of the heart rate signal received from the heart rate acquisition unit 16. Then, the fatigue degree estimation unit 22 is configured not to estimate the fatigue degree, when the heart rate increases after the vehicle starts the deceleration compared to the heart rate before the vehicle starts the deceleration and exceeds the preset heart rate stable value after the vehicle stops.

In this case, as the heart rate stable value, for example, an average value of the heart rate detected in the travelling state other than the deceleration state and the stopped state may be used. It is noted that the heart rate stable value may be set as a single value or as a range based on an upper limit and a lower limit of an interval in which the heart rate value keeps a similar value.

The fatigue degree notice unit 8 may be configured of a monitor and a speaker, for example. The fatigue degree notice unit 8 may be disposed at a position in a vehicle interior of the vehicle where the driver can hear a sound output from the fatigue degree notice unit 8 or where the driver can see an image displayed on the fatigue degree notice unit 8.

Furthermore, the fatigue degree notice unit 8 is configured to present at least one of image information displayed on the monitor and sound information output from the speaker to the driver of the vehicle depending on the notice command signal output from the main unit 6. In this way, the driver of the vehicle is encouraged to take a break or the attention of the driver is invited during travelling.

It is noted that the image information displayed on the monitor may be a still image including a message encouraging the driver to take a break or a message to invite attention of the driver during travelling. Furthermore, the sound information output from the speaker may be a sound message encouraging the driver to take a break or a sound message to invite attention of the driver during travelling.

(Processing Performed by Driver's Fatigue Degree Estimation Apparatus 1)

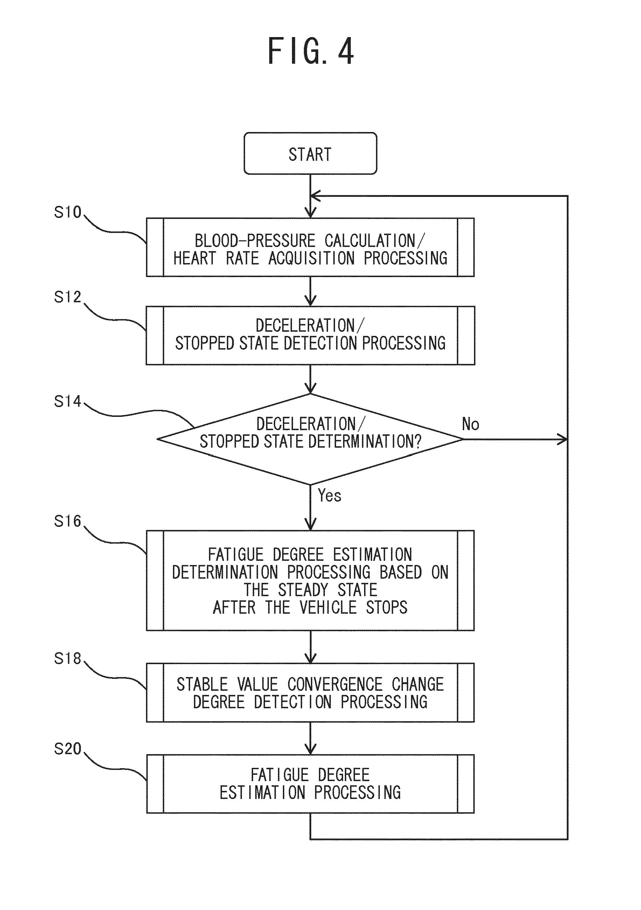

Hereinafter, a processing performed by the driver's fatigue degree estimation apparatus 1 of the present embodiment will be described with reference to FIG. 1 to FIG. 3A and FIG. 3B by using FIG. 4.

FIG. 4 is a flowchart illustrating one example of the processing performed by the driver's fatigue degree estimation apparatus 1.

As illustrated in FIG. 4, when the driver's fatigue degree estimation apparatus 1 starts the processing (START), firstly, the biological information value calculation unit 14 calculates the blood-pressure value of the driver on the basis of the electrocardiographic waveform signal and the pulse wave signal in the step S10. In addition, the heart rate acquisition unit 16 acquires the heart rate of the driver on the basis of the pulse wave signal. That is, the blood-pressure value of the driver is calculated and the heart rate of the driver is acquired ("blood-pressure calculation/heart rate acquisition processing" illustrated in the drawing) in the step S10. After the blood-pressure value of the driver is calculated and the heart rate of the driver is acquired in the step S10, the processing performed by the driver's fatigue degree estimation apparatus 1 proceeds to the step S12.

In the step S12, the deceleration/stopped state determination unit 18 detects the current vehicle speed on the basis of the vehicle speed signal in order to determine whether or not the state of the vehicle shifts from the deceleration state to the stopped state ("deceleration/stopped state detection processing" illustrated in the drawing). When the current vehicle speed is detected in the step S12, the processing performed by the driver's fatigue degree estimation apparatus 1 proceeds to the step S14.

In the step S14, it is determined whether or not the state of the vehicle shifts from the deceleration state to the stopped state on the basis of the vehicle speed detected in the step S12 ("deceleration/stopped state determination ?" illustrated in the drawing).

When it is determined that the state of the vehicle shifts from the deceleration state to the stopped state in the step S14 ("Yes" illustrated in the drawing), the processing performed by the driver' s fatigue degree estimation apparatus 1 proceeds to the step S16.

On the other hand, when it is determined that the state of the vehicle does not shift from the deceleration state to the stopped state in the step S14 ("No" illustrated in the drawing), the processing performed by the driver's fatigue degree estimation apparatus 1 returns to the step S10.

In the step S16, the fatigue degree estimation unit 22 compares the heart rate of the driver acquired in the step S10 with the heart rate stable value to determine whether or not the heart rate, which increases after the vehicle starts the deceleration compared to the heart rate before the vehicle starts the deceleration, exceeds the heart rate stable value after the vehicle stops.

Then, when the heart rate which increases after the vehicle starts the deceleration compared to the heart rate before the vehicle starts the deceleration exceeds the heart rate stable value after the vehicle stops, the processing performed by the driver's fatigue degree estimation apparatus 1 returns to the step S10. On the other hand, when the heart rate which increases after the vehicle starts the deceleration compared to the heart rate before the vehicle starts the deceleration does not exceed the heart rate stable value after the vehicle stops, the processing performed by the driver's fatigue degree estimation apparatus 1 proceeds to the step S18.

That is, in the step S16, the fatigue degree estimation unit 22 determines on the basis of the heart rate after the vehicle stops whether or not the processing for estimating the fatigue degree is to be performed ("fatigue degree estimation determination processing based on the steady state after the vehicle stops" illustrated in the drawing).

In the step S18, the stable value convergence change degree detection unit 20 detects the blood-pressure convergence change degree as the stable value convergence change degree on the basis of the blood-pressure value calculated in the step S10 and the vehicle speed detected in the step S12 ("stable value convergence change degree detection processing" illustrated in the drawing). When the stable value convergence change degree is detected in the step S18, the processing performed by the driver's fatigue degree estimation apparatus 1 proceeds to the step S20.

In the step S20, the fatigue degree estimation unit 22 performs the processing for estimating the fatigue degree of the driver on the basis of the stable value convergence change degree detected in the step S18 ("fatigue degree estimation processing" illustrated in the drawing). When the processing for estimate the fatigue degree of the driver is performed in the step S20, the processing performed by the driver's fatigue degree estimation apparatus 1 returns to the step S10.

(Operation)

Next, one example of the processing performed by the vehicle including the driver's fatigue degree estimation apparatus 1 of the present embodiment is described with reference to FIG. 1 to FIG. 4.

When driving the vehicle including the driver's fatigue degree estimation apparatus 1, the electrocardiogram sensor 10 detects the electrocardiographic waveform of the driver seated on the driver's seat and outputs the electrocardiographic waveform signal to the main unit 6. In addition, the pulse wave sensor 12 detects the pulse wave of the driver gripping the grip portion of the steering wheel and outputs the pulse wave signal to the main unit 6. Furthermore, the vehicle speed detection unit 4 detects the vehicle speed and outputs the vehicle speed signal to the main unit 6.

When the main unit 6 receives the electrocardiographic waveform signal, the pulse wave signal, and the vehicle speed signal, the biological information value calculation unit 14 calculates the blood-pressure value of the driver as the biological information value on the basis of the electrocardiographic waveform signal and the pulse wave signal, and outputs the blood-pressure value signal to the stable value convergence change degree detection unit 20. In addition, the deceleration/stopped state determination unit 18 determines the state of the vehicle on the basis of the vehicle speed signal, and output the determination result signal to the stable value convergence change degree detection unit 20. Furthermore, the heart rate acquisition unit 16 acquires the heart rate of the driver on the basis of the pulse wave signal, and outputs heart rate signal to the fatigue degree estimation unit 22.

When the stable value convergence change degree detection unit 20 receives the blood-pressure value signal and the determination result signal, the stable value convergence change degree detection unit 20 detects the blood-pressure convergence change degree as the stable value convergence change degree, and outputs the stable value convergence change degree signal to the fatigue degree estimation unit 22.

When the fatigue degree estimation unit 22 receives the heart rate signal, the fatigue degree estimation unit 22 compares the heart rate included in the heart rate signal with the heart rate stable value. Then, when the heart rate which increases after the vehicle starts the deceleration compared to the heart rate before the vehicle starts the deceleration does not exceed the heart rate stable value after the vehicle stops, the fatigue degree estimation unit 22 estimates the fatigue degree.

Then the fatigue degree estimation unit 22 estimates the fatigue degree of the driver on the basis of the stable value convergence change degree signal received in this way depending on the stable value convergence change degree included in the stable value convergence change degree signal. The fatigue degree estimation unit 22 outputs the notice command signal to the fatigue degree notice unit 8 depending on the magnitude of the estimated fatigue degree.

When the fatigue degree notice unit 8 receives the notice command signal, the fatigue degree notice unit 8 presents at least one of the image information displayed on the monitor and the sound information output from the speaker to the driver to encourage the driver to take a break or to invite the attention of the driver during travelling.

It is noted that the driver's fatigue degree estimation method performed in the operation of the driver's fatigue degree estimation apparatus 1 of the present embodiments is a method for estimating the fatigue degree of the driver depending on the detected stable value convergence change degree, as mentioned above. Herein, the stable value convergence change degree is a degree of the change in the biological information value from the peak value of the biological information value increasing after the vehicle starts the deceleration compared to the biological information value before the vehicle starts the deceleration, to the preset stable value, to which the biological information value decreases after the vehicle stops. The stable value convergence change degree is detected on the basis of the biological information value of the driver of the vehicle and the speed of the vehicle.

(Advantageous Effects of the First Embodiment)

The driver's fatigue degree estimation apparatus 1 of the present embodiment exhibits the following effects.

(1) The stable value convergence change degree detection unit 20 detects the stable value convergence change degree on the basis of the biological information value acquired by the biological information value acquisition unit 2 and the vehicle speed detected by the vehicle speed detection unit 4. Then, the fatigue degree estimation unit 22 estimates the fatigue degree of the driver depending on the stable value convergence change degree detected by the stable value convergence change degree detection unit 20.

Therefore, it is possible to estimate the fatigue degree of the driver on the basis of the degree of the change in the biological information value from the peak value of the biological information value increasing after the vehicle starts the deceleration compared to the biological information value before the vehicle starts the deceleration, to the stable value, to which the biological information value decreases after the vehicle stops.

As a result, it is possible to estimate the fatigue degree of the driver by using the biological information values at the times when the vehicle decelerates and stops regardless of the driving situation in regard to the road environment, the traffic environment or the like, and thus, it is possible to suppress the degradation of the estimate accuracy of the fatigue degree.

(2) The stable value convergence change degree detection unit 20 detects the deceleration stabilizing change rate, which is the change rate of the biological information value of the driver calculated on the basis of the deceleration stabilizing time and the deceleration stabilizing change amount, as the stable value convergence change degree.

Therefore, it is possible to detect the stable value convergence change degree on the basis of the an elapsed time and the change amount of the biological information value from when the biological information value starts to decrease from the peak value of the biological information value increasing after the vehicle starts the deceleration compared to the biological information value before the vehicle starts the deceleration, to when the biological information value reaches the stable value after the vehicle stops.

As a result, it is possible to estimate the fatigue degree of the driver depending on the change amount of the biological information value per unit time (for example, 1 (sec)), and thus, it is possible to suppress the degradation of the estimate accuracy of the fatigue degree.

(3) The biological information value acquisition unit 2 acquires the blood-pressure value of the driver as the biological information value of the driver. In addition, the stable value convergence change degree detection unit 20 detects the blood-pressure convergence change degree as the stable value convergence change degree. The blood-pressure convergence change degree is a degree of change in the blood-pressure value from the maximum blood-pressure value of the blood-pressure value rising after the vehicle starts the deceleration compared to the blood-pressure value before the vehicle starts the deceleration, to the stable blood-pressure value, to which the blood-pressure value drops after the vehicle stops.

Therefore, it is possible to estimate the fatigue degree of the driver on the basis of the degree of the change in the blood-pressure value, which is correlative with the fatigue degree of the driver, while the blood-pressure value reaches and then drops from the maximum blood-pressure value to the stable value (stable blood-pressure value).

As a result, it is possible to detect the electrocardiographic waveform and the pulse wave of the driver to estimate the fatigue degree of the driver, and thus, the estimation of the fatigue degree with a smaller load of the driver is possible.

(4) The heart rate acquisition unit 16 acquires the heart rate of the driver. In addition, the fatigue degree estimation unit 22 does not perform the processing for estimating the fatigue degree, when the heart rate of the driver which increases after the vehicle starts the deceleration compared to the heart rate before the vehicle starts the deceleration exceeds the preset heart rate stable value after the vehicle stops.

As a result, it is possible to eliminate a driver who is not in a steady state while the vehicle stops, that is, a driver unsuitable as an estimation target of the fatigue degree, from the estimation target of the fatigue degree, and thus, it is possible to suppress the degradation of the estimate accuracy of the fatigue degree.

(5) The fatigue degree estimation unit 22 is configured to estimate that the fatigue degree of the driver is lager as the stable value convergence change degree detected by the stable value convergence change degree detection unit 20 is smaller.

As a result, it is possible to estimate the fatigue degree of the driver on the basis of the magnitude of the stable value convergence change degree correlative with the fatigue degree of the driver, and thus, the load of the processing for estimating the fatigue degree can be reduced.

(6) The fatigue degree estimation unit 22 outputs the notice command signal to the fatigue degree notice unit 8 on the basis of the magnitude of the fatigue degree of the driver. Then, the fatigue degree notice unit 8 notifies the information indicative of the massage to encourage a break or an attention call to the driver.

As a result, it is possible to notify the information indicative of the massage to encourage a break or an attention call to the driver of the travelling vehicle depending on the fatigue degree, and thus, it is possible to suppress the occurrence of a minor collision or the like against another vehicle due to driving in the fatigued state.

(7) In the driver's fatigue degree estimation method of the present embodiment, the fatigue degree of the driver is estimated on the basis of the stable value convergence change degree which is a degree of change in the biological information value from a peak value of the biological information value increasing after the vehicle starts the deceleration compared to the biological information before the vehicle starts the deceleration, to the preset stable value, to which the biological information decreases after the vehicle stops.

Therefore, it is possible to estimate of the fatigue degree of the driver on the basis of the degree of the change in the biological information value from the peak value of the biological information value increasing after the vehicle starts the deceleration compared to the biological information value before the vehicle starts the deceleration, to the stable value, to which the biological information value decreases after the vehicle stops.

As a result, it is possible to estimate the fatigue degree of the driver by using the biological information values at the times when the vehicle decelerates and stops regardless of the driving situation in regard to the road environment, the traffic environment or the like, and thus, it is possible to suppress the degradation of the estimate accuracy of the fatigue degree.

(Modifications)

(1) In the present embodiment, the stable value convergence change degree detection unit 20 is configured to detect the deceleration stabilizing change rate as the stable value convergence change degree. However, the configuration of the stable value convergence change degree detection unit 20 is not limited thereto. That is, for example, the stable value convergence change degree detection unit 20 may be configured to detect a stop stabilizing change rate which is a change rate of the biological information value of the driver calculated on the basis of a stop stabilizing time and a stop stabilizing change amount, as the stable value convergence change degree.

Herein, the stop stabilizing time is a time which elapses while the vehicle stops, in an elapsed time from when the biological information value starts to decrease from the peak value of the biological information value increasing after the vehicle starts the deceleration compared to the biological information value before the vehicle starts the deceleration, to when the biological information value reaches the stable value after the vehicle stops. Furthermore, the stop stabilizing change amount is a change amount in the biological information value while the vehicle stops, of the change amount from the peak value of the biological information value increasing after the vehicle starts the deceleration compared to the biological information value before the vehicle starts the deceleration, to the stable value starts, to which the biological information value decreases after the vehicle stops.

In this case, it is possible to estimate the fatigue degree of the driver on the basis of the change rate and the change amount of the biological information value of the driver while the vehicle stops, and thus, it is possible to estimate the fatigue degree exclusive of the load of the driver due to a decelerating operation.

As a result, it is possible to estimate the fatigue degree during a general braking for a traffic signal or the like exclusive of braking due to a cause other than the change of the fatigue degree, such as emergency braking, and thus, it is possible to suppress the degradation of the estimate accuracy of the fatigue degree.

(2) In the present embodiment, the biological information value acquisition unit 2 is configured to acquire the blood-pressure value of the driver as the biological information value of the driver. However, the configuration of the biological information value acquisition unit 2 is not limited thereto. That is, the biological information value acquisition unit 2 may be configured to acquire a pulse pressure of the driver as the biological information value of the driver. In this case, the stable value convergence change degree detection unit 20 is configured to detect a pulse pressure convergence change degree as the stable value convergence change degree.

Herein, the pulse pressure convergence change degree is a degree of change in the pulse pressure from a minimum pulse pressure of the pulse pressure narrowing after the vehicle starts the deceleration compared to the pulse pressure before the vehicle starts the deceleration, to a preset stable pulse pressure, to which the pulse pressure widens after the vehicle stops.

It is noted that, the minimum pulse pressure corresponds to the peak of the above mentioned biological information, and thus, may be a minimum value of the pulse pressure. Furthermore, when the pulse pressure fluctuates greatly or the like, an average value in an interval in which the pulse pressure keeps a similar value may be used.

Furthermore, an average value of the pulse pressure detected in a travelling state other than the deceleration state and the stopped state may be used as the stable pulse pressure, for example. It is noted that the stable pulse pressure may be set as a single value or as a range based on an upper limit and a lower limit of the interval in which the pulse pressure keeps a similar value.

In such a configuration, it is possible to estimate the fatigue degree of the driver on the basis of the degree of change in the pulse pressure correlative with the fatigue degree of the driver, while the pulse pressure narrows compared to the pulse pressure before the vehicle starts the deceleration and such a narrowed pulse pressure widens and reaches the stable value (stable pulse pressure). It is noted that the pulse pressure has a certain correlation with the blood-pressure value, and such a configuration may use a fingertip pulse pressure measurable more easily than the blood-pressure value.

As a result, it is possible to detect the pulse pressure of the driver to estimate the fatigue degree of the driver, and thus, the estimation of the fatigue degree with a smaller load of the driver is possible.

(3) In the present embodiment, the biological information value acquisition unit 2 is configured to acquire the blood-pressure value of the driver as the biological information of the driver, the configuration of the biological information value acquisition unit 2 is not limited thereto. That is, the biological information value acquisition unit 2 may be configured to acquire the pulse transmission time of the driver as the biological information of the driver. In this case, the stable value convergence change degree detection unit 20 is configured to detect a pulse transmission time convergence change degree as the stable value convergence change degree.

Herein, the pulse transmission time convergence change degree is a degree of change in the pulse transmission time from a maximum pulse transmission time of the pulse transmission time increasing after the vehicle starts the deceleration compared to the pulse transmission time before the vehicle starts the deceleration, to a preset stable pulse transmission time, to which the pulse transmission time decreases after the vehicle stops.

It is noted that the minimum pulse transmission time corresponds to the peak of the above mentioned biological information, and thus, may be a maximum value of the pulse transmission time. Furthermore, when the pulse transmission time fluctuates greatly or the like, an average value in an interval in which the pulse transmission time keeps a similar value may be used.

Furthermore, an average value of the pulse transmission time detected in a travelling state other than the deceleration state and the stopped state may be used as the stable pulse transmission time, for example. It is noted that the stable pulse transmission time may be set as a single value or as a range based on an upper limit and a lower limit of the interval in which the pulse transmission time keeps a similar value.

In such a configuration, it is possible to estimate the fatigue degree of the driver on the basis of the degree of change in the pulse transmission time correlative with the fatigue degree of the driver, from the pulse transmission time decreasing compared to before the vehicle starts the deceleration, to the stable value (stable pulse transmission time), to which the pulse transmission time increases.

As a result, it is possible to detect the pulse transmission time of the driver to estimate the fatigue degree of the driver, and thus, the estimation of the fatigue degree with a smaller load of the driver is possible.

(4) In the present embodiment, the fatigue degree estimation unit 22 is configured not to estimate the fatigue degree when the heart rate increases after the vehicle starts the deceleration compared to the heart rate before the vehicle starts the deceleration and exceeds the heart rate stable value after the vehicle stops. However, the configuration of the fatigue degree estimation unit 22 is not limited thereto. Thus, the fatigue degree estimation unit 22 may be configured to estimate the fatigue degree regardless of the heart rate of the driver.

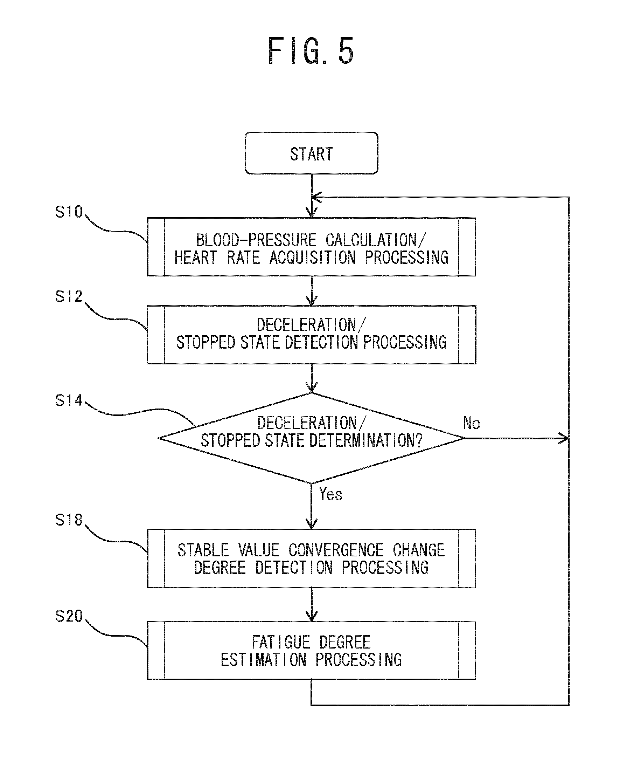

In this case, the processing performed by the driver's fatigue degree estimation apparatus 1 is changed such that a processing in the step S16 is eliminated from the processing performed by the driver's fatigue degree estimation apparatus 1 of the present embodiment, as illustrated in FIG. 5. It is noted that FIG. 5 is a flowchart illustrating one example of a processing performed by the modification of a driver's fatigue degree estimation apparatus.

(5) In the present embodiment, the configuration of the driver's fatigue degree estimation apparatus 1 includes the fatigue degree notice unit 8. However, the driver's fatigue degree estimation apparatus 1 is not limited thereto, and may have a configuration without the fatigue degree notice unit 8.

Priority is claimed on Japanese Patent Application No. 2012-166227 (filed on July 26, 2012), the entire content of which is incorporated by reference as a part of this application.

While the present invention has been described with reference to the definite number of embodiments, the scope of the present invention is not limited thereto and improvements and modifications of the embodiments based on the above disclosure are obvious to those skilled in the art.

Reference Signs List

1    driver's fatigue degree estimation apparatus
2    biological information value acquisition unit
4    vehicle speed detection unit
6    main unit

8    fatigue degree notification unit
10   electrocardiogram sensor
12   pulse wave sensor
14   biological information value calculation unit
16   heart rate acquisition unit
18   deceleration/stopped state determination unit
20   stable value convergence change degree detection unit
22   fatigue degree estimation unit

**Claims**

1.  A driver's fatigue degree estimation apparatus comprising:

a biological information value acquisition unit configured to acquire a biological information value indicative of biological information of a driver of a vehicle;
a vehicle speed detection unit configured to detect a vehicle speed of the vehicle;
a stable value convergence change degree detection unit configured to detect a stable value convergence change degree which is a degree of change in the biological information value from a peak value of the biological information value increasing after the vehicle starts deceleration compared to the biological information value before the vehicle starts the deceleration, to a preset stable value, to which the biological information value decreases after the vehicle stops, based on the biological information value acquired by the biological information value acquisition unit and the vehicle speed detected by the vehicle speed detection unit; and
a fatigue degree estimation unit configured to estimate a fatigue degree of the driver depending on the stable value convergence change degree detected by the stable value convergence change degree detection unit.

2.  The driver's fatigue degree estimation apparatus according to claim 1, wherein
the stable value convergence change degree detection unit is configured to detect a deceleration stabilizing change rate as the stable value convergence change degree based on the biological information value acquired by the biological information value acquisition unit and the vehicle speed detected by the vehicle speed detection unit,
the deceleration stabilizing change rate is a change rate of the biological information value calculated based on a deceleration stabilizing time and a deceleration stabilizing change amount,
the deceleration stabilizing time is an elapsed time from when the biological information value starts to decrease from the peak value of the biological information value increasing after the vehicle starts the deceleration compared to the biological information value before the vehicle starts the deceleration, to when the biological information value reaches the stable value after the vehicle stops,
the deceleration stabilizing change amount is a change amount in the biological information from the peak value of the biological information value increasing after the vehicle starts the deceleration compared to the biological information value before the vehicle starts the deceleration, to the preset stable value, to which the biological information value decreases after the vehicle stops.

3. The driver's fatigue degree estimation apparatus according to claim 1, wherein
the stable value convergence change degree detection unit is configured to detect a stop stabilizing change rate as the stable value convergence change degree based on the biological information value acquired by the biological information value acquisition unit and the vehicle speed detected by the vehicle speed detection unit,
the stop stabilizing change rate is a change rate of the biological information value calculated based on a stop stabilizing time and a stop stabilizing change amount,
the stop stabilizing time is a time which elapses while the vehicle stops in a elapsed time from when the biological information value starts to decrease from the peak value of the biological information value increasing after the vehicle starts the deceleration compared to the biological information value before the vehicle starts the deceleration, to when the biological information value reaches the stable value after the vehicle stops, and
the stop stabilizing change amount is a change amount in the biological information value while the vehicle stops of the change amount from the peak value of the biological information value increasing after the vehicle starts the deceleration compared to the biological information value before the vehicle starts the deceleration, to the preset stable value, to which the biological information value decreases after the vehicle stops.

4. The driver's fatigue degree estimation apparatus according to any one of claims 1 to 3, wherein
the biological information value acquisition unit is configured to acquire a blood-pressure value of the driver as the biological information value,
the stable value convergence change degree detection unit is configured to detect a blood-pressure convergence change degree as the stable value convergence change degree based on the blood-pressure value acquired by the biological information value acquisition unit and the vehicle speed detected by the vehicle speed detection unit, and

the blood-pressure convergence change degree is a degree of change in the blood-pressure value from a maximum blood-pressure value of the blood-pressure value rising after the vehicle starts the deceleration compared to the blood-pressure value before the vehicle starts the deceleration, to a preset stable blood-pressure value, to which the blood-pressure value drops after the vehicle stops.

5. The driver's fatigue degree estimation apparatus according to any one of claims 1 to 3, wherein
the biological information value acquisition unit is configured to acquire a pulse pressure of the driver as the biological information value,
the stable value convergence change degree detection unit is configured to detect a pulse pressure convergence change degree as the stable value convergence change degree based on the pulse pressure acquired by the biological information value acquisition unit and the vehicle speed detected by the vehicle speed detection unit, and
the pulse pressure convergence change degree is a degree of change in the pulse pressure from a minimum pulse pressure of the pulse pressure narrowing after the vehicle starts the deceleration compared to the pulse pressure before the vehicle starts the deceleration, to a preset stable pulse pressure, to which the pulse pressure widens after the vehicle stops.

6. The driver's fatigue degree estimation apparatus according to any one of claims 1 to 3, wherein
the biological information value acquisition unit is configured to acquire a pulse transmission time of the driver as the biological information value,
the stable value convergence change degree detection unit is configured to detect a pulse transmission time convergence change degree as the stable value convergence change degree based on the pulse transmission time acquired by the biological information value acquisition unit and the vehicle speed detected by the vehicle speed detection unit, and
the pulse transmission time convergence change degree is a degree of change in the pulse transmission time from a maximum pulse transmission time of the pulse transmission time increasing after the vehicle starts the deceleration compared to the pulse transmission time before the vehicle starts the deceleration, to a preset stable pulse transmission time, to which the pulse transmission time decreases after the vehicle stops.

7. The driver's fatigue degree estimation apparatus according to any one of claims 1 to 6, further comprising a heart rate acquisition unit configured to acquire a heart rate of the driver,
wherein the fatigue degree estimation unit is configured not to estimate the fatigue degree when the heart rate increases after the vehicle starts the deceleration compared to the heart rate before the vehicle starts the deceleration and exceeds a preset heart rate stable value after the vehicle stops.

8. The driver's fatigue degree estimation apparatus according to any one of claims 1 to 7, wherein the fatigue degree estimation unit is configured to estimate that the fatigue degree is lager as the stable value convergence change degree detected by the stable value convergence change degree detection unit is smaller.

9. A driver's fatigue degree estimation method comprising:

detecting a stable value convergence change degree based on a biological information value indicative of biological information of a driver of a vehicle and a vehicle speed of the vehicle, the stable value convergence change degree being a degree of change in the biological information value from a peak value of the biological information value increasing after the vehicle starts deceleration compared to the biological information value before the vehicle starts the deceleration, to a preset stable value, to which the biological information value decreases after the vehicle stops, and
estimating a fatigue degree of the driver depending on the stable value convergence change degree.

# FIG. 1

# F I G. 2

FIG. 3A

FIG. 3B

START OF DECELERATION    STOPPED

START OF DECELERATION    STOPPED

EP 2 878 262 A1

# FIG. 4

START

S10 — BLOOD-PRESSURE CALCULATION/
HEART RATE ACQUISITION PROCESSING

S12 — DECELERATION/
STOPPED STATE DETECTION PROCESSING

S14 — DECELERATION/
STOPPED STATE DETERMINATION?  →  No

Yes

S16 — FATIGUE DEGREE ESTIMATION
DETERMINATION PROCESSING BASED ON
THE STEADY STATE
AFTER THE VEHICLE STOPS

S18 — STABLE VALUE CONVERGENCE CHANGE
DEGREE DETECTION PROCESSING

S20 — FATIGUE DEGREE
ESTIMATION PROCESSING

# FIG. 5

START

S10 — BLOOD-PRESSURE CALCULATION/
HEART RATE ACQUISITION PROCESSING

S12 — DECELERATION/
STOPPED STATE DETECTION PROCESSING

S14 — DECELERATION/
STOPPED STATE DETERMINATION?　　　No

Yes

S18 — STABLE VALUE CONVERGENCE CHANGE
DEGREE DETECTION PROCESSING

S20 — FATIGUE DEGREE
ESTIMATION PROCESSING

<div style="text-align:center">**INTERNATIONAL SEARCH REPORT**</div>

| International application No. |
|---|
| PCT/JP2013/004512 |

**A. CLASSIFICATION OF SUBJECT MATTER**
*A61B5/18*(2006.01)i, *A61B5/16*(2006.01)i, *B60K28/06*(2006.01)i, *B60W30/08* (2012.01)i, *B60W50/08*(2012.01)i

According to International Patent Classification (IPC) or to both national classification and IPC

**B. FIELDS SEARCHED**

Minimum documentation searched (classification system followed by classification symbols)
A61B5/18, A61B5/16, B60K28/06, B60W30/08, B60W50/08

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2002-65650 A (Nissan Motor Co., Ltd.), 05 March 2002 (05.03.2002), entire text; all drawings (Family: none) | 1-9 |
| A | JP 2009-146185 A (Fujitsu Ten Ltd.), 02 July 2009 (02.07.2009), entire text; all drawings (Family: none) | 1-9 |
| A | JP 2010-63682 A (Aisin Seiki Co., Ltd.), 25 March 2010 (25.03.2010), entire text; all drawings (Family: none) | 1-9 |

☐ Further documents are listed in the continuation of Box C.       ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 August, 2013 (14.08.13) | 27 August, 2013 (27.08.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007038911 A
- JP 2012166227 A